(19) **Europäisches Patentamt**

**European Patent Office**

**Office européen des brevets**

(11) **EP 0 797 991 B1**

(12) ## EUROPEAN PATENT SPECIFICATION

(45) Date of publication and mention
of the grant of the patent:
**02.01.2004  Bulletin 2004/01**

(51) Int Cl.⁷: **A61K 31/135**, A61K 9/50

(21) Application number: **97301937.5**

(22) Date of filing: **21.03.1997**

(54) **Extended release formulation containing venlafaxine**

Arzneimittel mit verzögerter Wirkstoffabgabe enthaltend Venlafaxin

Compositions de venlafaxine à libération prolongée

(84) Designated Contracting States:
**AT BE CH DE DK ES FI FR GB GR IE IT LI LU NL PT SE**
Designated Extension States:
**AL LT LV RO SI**

(30) Priority: **25.03.1996  US 14006**

(43) Date of publication of application:
**01.10.1997  Bulletin 1997/40**

(60) Divisional application:
**03008911.4 / 1 331 003**

(73) Proprietor: **Wyeth**
**Madison, New Jersey 07940-0874 (US)**

(72) Inventors:
• **Sherman, Deborah Marie**
**Plattsburgh, New York 12901 (US)**

• **CLARK John Clifton**
**New York 12972 (US)**
• **LAMER John Ulrick**
**Vermont, 05478 (US)**
• **WHITE Stephen Andrew**
**New York 12979 (US)**

(74) Representative: **Connelly, Michael John et al**
**c/o Patent Department**
**Wyeth Laboratories**
**Huntercombe Lane South**
**Taplow**
**Maidenhead Berkshire SL6 0PH (GB)**

(56) References cited:
**EP-A- 0 112 669      EP-A- 0 639 374**
**EP-A- 0 654 264      WO-A-94/27589**

EP 0 797 991 B1

**Description**

Background of the invention

Extended release drug formulations are conventionally produced as compressed tablets by hydrogel tablet technology. To produce these sustained release tablet drug dosage forms, the active ingredient is conventionally compounded with cellulose ethers such as methyl cellulose, ethyl cellulose or hydroxypropylmethylcellulose with or without other excipients and the resulting mixture is pressed into tablets. When the tablets are orally administered, the cellulose ethers in the tablets swell upon hydration from moisture in the digestive system, thereby limiting exposure of the active ingredient to moisture. As the cellulose ethers are gradually leached away by moisture, water more deeply penetrates the gel matrix and the active ingredient slowly dissolves and diffuses through the gel, making it available for absorption by the body. An example of such a sustained release dosage form of the analgesic/antiinflammatory drug etodolac (Lodine®) appears in US patent 4,966,768.

Where the production of tablets is not feasible, it is conventional in the drug industry to prepare encapsulated drug formulations which provide extended or sustained release properties. In this situation, the extended release capsule dosage forms may be formulated by mixing the drug with one or more binding agents to form a uniform mixture which is then moistened with water or a solvent such as ethanol to form an extrudable plastic mass from which small diameter, typically 1 mm, cylinders of drug/matrix are extruded, chopped into appropriate lengths and transformed into spheroids using standard spheronization equipment. The spheroids, after drying, may then be film-coated to retard dissolution. Gelatin capsules are filled with the film-coated spheroids in the quantity needed to obtain the desired therapeutic effect. Spheroids releasing the drug at different rates may be combined in a gelatin capsule to obtain desired release rates and blood levels. US patent 4,138,475 discloses a sustained release pharmaceutical composition consisting of a hard gelatin capsule filled with film-coated spheroids comprised of propanolol in admixture with microcrystalline cellulose wherein the film coating is composed of ethyl cellulose, optionally with hydroxypropylmethylcellulose and/or a plasticizer.

Venlafaxine, 1-[2-(dimethylamino)-1-(4-methoxyphenyl)ethyl]cyclohexanol, is an important drug in the neuropharmacological arsenal used for treatment of depression. Venlafaxine and the acid addition salts thereof are disclosed in US patent 4,535,186. Venlafaxine hydrochloride is presently administered to adults in compressed tablet form in doses ranging from 75 to 350 mg/day, in divided doses two or three times a day. In therapeutic dosing with venlafaxine hydrochloride tablets, rapid dissolution results in a rapid increase in blood plasma levels of the active compound shortly after administration followed by a decrease in blood plasma levels over several hours as the active compound is eliminated or metabolized, until sub-therapeutic plasma levels are approached after about twelve hours following administration, thus requiring additional dosing with the drug. With the plural daily dosing regimen, the most common side effect is nausea, experienced by about forty five percent of patients under treatment with venlafaxine hydrochloride. Vomiting also occurs in about seventeen percent of the patients.

WO 94 27589 A discloses a dosage form for venlafaxine, comprising a wall, a compartment comprising the drug, a displacement agent and an exit passage way.

Brief Description of the Invention

In accordance with this invention, there is provided an extended release (ER), encapsulated formulation containing venlafaxine hydrochloride as the active drug component, which provides in a single dose, a therapeutic blood serum level over a twenty four hour period. The invention particularly provides an encapsulated, extended release formulation of venlafaxine hydrochloride comprising a hard gelatin capsule containing a therapeutically effective amount of spheroids comprised of venlafaxine hydrochloride, microcrystalline cellulose and hydroxypropyl methylcellulose coated with ethyl cellulose and hydroxypropylmethylcellulose. The invention also provides an extended release formulation of venlafaxine hydrochloride for once daily administration which comprises spheroids containing 37.3% venlafaxine, 62.17% microcrystalline cellulose and 0.5% hydroxypropylmethylcellulose type 2208, coated with a quantity of a mixture comprised of 15% ethyl cellulose type HG 2834 and 85% hydroxypropylmethylcellulose type 2910 sufficient to give coated spheroids having a dissolution profile which gives the desired release rate over a 24 hour period. The invention also provides a coated spheroid in which the spheroid is comprised of venlafaxine hydrochloride, microcrystalline cellulose and hydroxypropyl methylcellulose and is coated with ethyl cellulose and hydroxypropylmethylcellulose.

Through administration of the venlafaxine formulation of this invention, there is obtained a method for obtaining a flattened drug plasma concentration to time profile, thereby affording a tighter plasma therapeutic range control than can be obtained with multiple daily dosing. In other words, this invention enables one to eliminate the sharp peaks and troughs (hills and valleys) in blood plasma drug levels induced by multiple daily dosing with conventional immediate release venlafaxine hydrochloride tablets. In essence, the plasma levels of venlafaxine hydrochloride rise, after administration of the extended release formulations of this invention, for between about five to about eight hours (optimally about six hours) and then begin to fall through a protracted, substantially linear decrease from the peak plasma level for the remainder of the twenty four hour period, maintaining at least a threshold therapeutic level of the drug during the entire twenty-four period. In contrast, the conventional immediate release venlafaxine hydrochloride tablets give peak blood plasma levels in 2 to 4 hours. Hence, one can moderate the plural blood plasma peaks and valleys attending the pharmacokinetic utilization of multiple daily tablet dosing with venlafaxine hydrochloride by administering to a patient in need of treatment with venlafaxine hydrochloride, a one-a-day, extended release formulation of venlafaxine hydrochloride.

The use of the one-a-day venlafaxine hydrochloride formulations of this invention reduces by adaptation, the level of nausea and incidence of emesis that attend the administration of multiple daily dosing. In clinical trials of venlafaxine hydrochloride ER, the probability of developing nausea in the course of the trials was greatly reduced after the first week. Venlafaxine ER showed a statistically significant improvement over conventional venlafaxine hydrochloride tablets in two eight-week and one 12 week clinical studies. Thus, one can reduce the level of nausea and incidence of emesis attending the administration of venlafaxine hydrochloride by dosing a patient in need of treatment with venlafaxine hydrochloride with an extended release formulation of venlafaxine hydrochloride once a day in a therapeutically effective amount.

Detailed Description of the Invention

1-[2-(dimethylamino)-1-(4-methoxyphenyl)ethyl]cyclohexanol hydrochloride is polymorphic. Of the forms isolated and characterized to date, Form I is considered to be the kinetic product of crystallization which can be converted to Form II upon heating in the crystallization solvent. Forms I and II cannot be distinguished by their melting points but do exhibit some differences in their infrared spectra and X-ray diffraction patterns. Any of the polymorphic forms such as Form I or Form II may be used in the formulations of the present invention.

The extended release formulations of this invention are comprised of 1-[2-(dimethylamino)-1-(4-methoxyphenyl)ethyl] cyclohexanol hydrochloride in admixture with microcrystalline cellulose and hydroxypropylmethylcellulose. Formed as beads or spheroids, the drug containing formulation is coated with a mixture of ethyl cellulose and hydroxypropylmethyl cellulose to provide the desired level of coating, generally from about two to about twelve percent on a weight/weight basis of final product or more preferably from about five to about ten percent (w/w), with best results obtained at from about 6 to about 8 percent (w/w). More specifically, the extended release spheroid formulations of this invention comprise from about 30 to 40 percent venlafaxine hydrochloride, from about 50 to about 70 percent microcrystalline cellulose, NF, from about 0.25 to about 1 percent hydroxypropylmethylcellulose, USP, and from about 5 to about 10 percent film coating, all on a weight/weight basis. And preferably, the spheroid formulations contain about 35 percent venlafaxine hydrochloride, about 55 to 60 percent microcrystalline cellulose NF (Avicel® PH101), about one half percent hydroxypropyl methylcellulose 2208 USP (K3, Dow, which has a viscosity of 3 cps for 2% aqueous solutions, a methoxy content of 19-24% and a hydroxypropoxy content of 4-13%), and from about 6 to 8 percent film coating.

The film coating is comprised of 80 to 90 percent of ethyl cellulose, NF and 10 to 20 percent hydroxypropyl methylcellulose (2910), USP on a weight/weight basis. Preferably the ethyl cellulose has a ethoxy content of 44.0-51% and a viscosity of 50 cps for a 5% aqueous solution and the hydroxypropylmethylcellulose is USP 2910 having a viscosity of 6 cps at 2% aqueous solution with a methoxy content of 28-30% and a hydroxypropoxy content of 7-12%. The ethyl cellulose used herein is Aqualon HG 2834.

The hydroxypropylmethylcelluloses 2208 and 2910 USP and ethyl cellulose, NF may be replaced in the formulation by another hydroxypropylmethylcellulose or ethyl cellulose having the same chemical and physical characteristics as the proprietary products named above.

It was completely unexpected that an extended release formulation containing venlafaxine hydrochloride could be obtained because the hydrochloride of venlafaxine proved to be extremely water soluble. Numerous attempts to produce extended release tablets by hydrogel technology proved to be fruitless because the compressed tablets were either physically unstable (poor compressibility or capping problems) or dissolved too rapidly in dissolution studies. Typically, the tablets prepared as hydrogel sustained release formulations gave 40-50% dissolution at 2 hrs, 60-70% dissolution at 4 hrs and 85-100% dissolution at 8 hrs.

Numerous spheroid formulations were prepared using different grades of microcrystalline cellulose and hydroxypropyl methylcellulose, different ratios of venlafaxine hydrochloride and filler, different binders such as polyvinylpyrrolidone, methylcellulose, water, and polyethylene glycol of different molecular weight ranges in order to find a formulation which would provide a suitable granulation mix which could be extruded properly. In the extrusion process, heat buildup occurred which dried out the extrudate so much that it was difficult to convert the extruded cylinders into spheroids. Addition of hydroxypropylmethylcellulose 2208 to the venlafaxine hydrochloride-microcrystalline cellulose mix made production of spheroids practical.

The following examples are presented to illustrate applicant's solution to the problem of preparation of the extended release drug containing formulations of this invention.

Example 1.

VENLAFAXINE HYDROCHLORIDE EXTENDED RELEASE CAPSULES

A mixture of 44.8 pans ( 88.4 % free base) of venlafaxine hydrochloride, 74.6 parts of the microcrystalline cellulose, NF, and 0.60 parts of hydroxypropylmethyl cellulose 2208, USP, are blended with the addition of 41.0 parts water. The plastic mass of material is extruded, spheronized and dried to provide uncoated drug containing spheroids.

Stir 38.25 parts of ethyl cellulose, NF, HG2834 and 6.75 parts of hydroxypropyl methylcellulose 2910, USP in a 1:1 v/v mixture of methylene chloride and anhydrous methanol until solution of the film coating material is complete.

To a fluidized bed of the uncoated spheroids is applied 0.667 parts of coating solution per part of uncoated spheroids to obtain extended release, film coated spheroids having a coating level of 3%.

The spheroids are sieved to retain the coated spheroids of a particle size between 0.85 mm to 1.76 mm diameter. These selected film coated spheroids are filled into hard gelatin capsules conventionally.

Example 2.

Same as for Example 1 except that 1.11 parts of the film coating solution per part of uncoated spheroids is applied to obtain a coating level of 5%.

Example 3.

Same as for Example 1 except that 1.33 parts of the film coating solution is applied to 1 part of uncoated spheroids to obtain a coating level of 6%.

Example 4.

Same as for Example 1 except that 1.55 pans of the film coating solution is applied to 1 part of uncoated spheroids to obtain a coating level of 7%.

The test for acceptability of the coating level is determined by analysis of the dissolution rate of the finished coated spheroids prior the encapsulation. The dissolution procedure followed uses USP Apparatus 1 (basket) at 100 rpm in purified water at 37°C. Conformance with the dissolution rate given in Table 1 provides the twenty-four hour therapeutic blood levels for the drug component of the extended release capsules of this invention in capsule form. Where a given batch of coated spheroids releases drug too slowly to comply with the desired dissolution rate study, a portion of uncoated spheroids or spheroids with a lower coating level may be added to the batch to provide, after thorough mixing, a loading dose for rapid increase of blood drug levels. A batch of coated spheroids that releases the drug too rapidly can receive additional film-coating to give the desired dissolution profile.

Table 1

| Acceptable Coated Spheroid Dissolution Rates | |
|---|---|
| Time (hours) | Average % Venlafaxine HCL released |
| 2 | <30 |
| 4 | 30-55 |
| 8 | 55-80 |
| 12 | 65-90 |
| 24 | >80 |

Batches of the coated venlafaxine hydrochloride containing spheroids which have a dissolution rate corresponding to that of Table 1 are filled into hard gelatin capsules in an amount needed to provide the unit dosage level desired. The standard unit dosage immediate release (IR) tablet used presently provides amounts of venlafaxine hydrochloride equivalent to 25 mg, 37.5 mg, 50 mg, 75 mg and 100 mg venlafaxine. The capsules of this invention are filled to provide an amount of venlafaxine hydrochloride equivalent to that presently used in tablet form and also up to about 150 mg venlafaxine hydrochloride.

Dissolution of the venlafaxine hydrochloride ER capsules is determined as directed in the U. S. Pharmacopoeia (USP) using apparatus 1 at 100 rpm on 0.9 L of water. A filtered sample of the dissolution medium is taken at the times specified. The absorbance of the clear solution is determined from 240 to 450 nanometers (nm) against the dissolution medium. A baseline is drawn from 450 nm through 400 nm and extended to 240 nm. The absorbance at the wavelength of maximum absorbance (about 274 nm) is determined with respect to this baseline. Six hard gelatin capsules are filled with the theoretical amount of venlafaxine hydrochloride spheroids and measured for dissolution. Standard samples consist of venlafaxine hydrochloride standard solutions plus a gelatin capsule correction solution. The percentage of venlafaxine released is determined from the equation

$$\% \text{ Venlafaxine hydrochloride released} = \frac{(As)(Wr)(S)(V1)(0.888)(100)}{(Ar)(V2)(C)}$$

where As is absorbance of sample preparation, Wr is weight of reference standard, mg; S is strength of the reference standard, decimal; V I is the volume of dissolution medium used to dissolve the dosage form, mL; 0.884 is the percent free base, Ar is the absorbance of the standard preparation, V2 is the volume of reference standard solution, mL; and C is the capsule claim in mg.

Table 2 shows the plasma level of venlafaxine versus time for one 75 mg conventional Immediate Release (IR) tablet administered every 12 hours, two 75 mg extended release (ER) capsules administered simultaneously every 24 hours, and one 150 mg extended release (ER) capsule administered once every 24 hours in human male subjects. The subjects were already receiving venlafaxine hydrochloride according to the dosage protocol, thus the plasma blood level at zero time when dosages were administered is not zero.

Table 2

| Plasma venlafaxine level (ng/mL) versus time, conventional tablet (not extended release) versus ER capsule | | | |
|---|---|---|---|
| Time (hours) | 75 mg (IR)tablet | 2 x 75 mg (ER)capsules | 1 x 150 mg (ER)capsules |
| | (q 12 h) | (q 24 hr) | (q 24 h) |
| 0 | 62.3 | 55.0 | 55.8 |
| 0.5 | 76.3 | | |
| 1 | 135.6 | 53.3 | 53.2 |
| 2 | 212.1 | 69.8 | 70.9 |
| 4 | 162.0 | 138.6 | 133.3 |
| 6 | 114.6 | 149.0 | 143.5 |
| 8 | 86.7 | 129.3 | 129.5 |
| 10 | | 118.4 | 114.4 |
| 12 | 51.9 | 105.1 | 105.8 |
| 12.5 | 74.7 | | |
| 13 | 127.5 | | |
| 14 | 161.3 | 90.5 | 91.3 |
| 16 | 134.6 | 78.2 | 78.5 |
| 18 | 106.2 | | |
| 20 | 83.6 | 62.7 | 63.3 |
| 24 | 57.6 | 56.0 | 57.3 |

Table 2 shows that the plasma levels of two 75 mg/capsule venlafaxine hydrochloride ER capsules and one 150 mg/capsule venlafaxine hydrochloride ER capsule provide very similar blood levels. The data also show that the plasma level after 24 hours for either extended release regimen is very similar to that provided by two immediate release 75 mg tablets of venlafaxine hydrochloride administered at 12 hour intervals.

Further, the plasma levels of venlafaxine obtained with the extended release formulation do not increase to the peak levels obtained with the conventional immediate release tablets given 12 hours apart. The peak level of venlafaxine from (ER) , somewhat below 150 ng/ml, is reached in about six hours, plus or minus two hours, based upon this specific dose when administered to patients presently under treatment with venlafaxine hydrochloride (IR). The peak plasma level of venlafaxine, somewhat over 200 ng/ml, following administration of (IR) is reached in two hours and falls rapidly thereafter.

Table 3 shows venlafaxine blood plasma levels in male human subjects having a zero initial blood plasma level. Again, a peak blood plasma concentration of venlafaxine is seen at about 6 hours after dosing with venlafaxine hydrochloride extended release capsules in the quantities indicated. The subjects receiving the single 50 mg immediate release tablet showed a peak plasma level occurring at about 4 hours. For comparative purposes, the plasma levels of venlafaxine for subjects receiving the conventional formulated tablet can be multiplied by a factor of three to approximate the plasma levels expected for a single dose of 150 mg. conventional formulation.

Table 3.

| Plasma Blood Levels in Human Males Having No Prior Venlafaxine Blood Level | | | |
|---|---|---|---|
| Time (Hours) | 1 x 50 mg IR tablet | 2 x 75 mg ER capsules | 1 x 150 mg ER capsule |
| 0 | 0 | 0 | 0 |
| 1 | 27.87 | 1.3 | 0 |
| 1.5 | 44.12 | 6.0 | 2.2 |
| 2 | 54.83 | 20.6 | 12.8 |
| 4 | 66.38 | 77.0 | 81.0 |
| 6 | 49.36 | 96.5 | 94.4 |
| 8 | 30.06 | 93.3 | 86.9 |
| 10 | 21.84 | 73.2 | 72.8 |
| 12 | 15.91 | 61.3 | 61.4 |
| 14 | 13.73 | 52.9 | 51.9 |
| 16 | 10.67 | 47.5 | 41.1 |
| 20 | 5.52 | 35.2 | 34.0 |
| 24 | 3.56 | 29.3 | 28.5 |
| 28 | 2.53 | 23.4 | 22.9 |
| 36 | 1.44 | 11.9 | 13.5 |
| 48 | 0.66 | 5.8 | 5.2 |

The blood plasma levels of venlafaxine were measured according to the following procedure. Blood samples from the subjects were collected in heparinized evacuated blood tubes and the tubes were inverted gently several times. As quickly as possible, the tubes were centrifuged at 2500 rpm for 15 minutes. The plasma was pipetted into plastic tubes and stored at -20°C until analysis could be completed.

To 1 mL of each plasma sample in a plastic tube was added 150 μL of a stock infernal standard solution (150 μg/ml). Saturated sodium borate (0.2 mL) solution was added 10 each tube and vortexed. Five mL of ethyl ether was added to each tube which were then capped and shaken for 10 minutes at high speed. The tubes were centrifuged at 3000 rpm for 5 minutes. The aqueous layer was frozen in dry ice and the organic layer transferred to a clean screw cap tube. A 0.3 mL portion of 0.01 N HCl solution was added to each tube and shaken for 10 minutes at high speed. The aqueous layer was frozen and the organic layer removed and discarded. A 50μL portion of the mobile phase (23: 77 acetonitrile:0.1M monobasic ammonium phosphate buffer, pH 4.4) was added to each tube, vonexed, and 50 μL samples were injected on a Supelco Supelcoil LC-8-DB, 5 cm x 4.6 mm, 5 μ column in a high pressure liquid chromatography apparatus equipped with a Waters Lambda Max 481 detector or equivalent at 229 nm. Solutions of venlafaxine hydrochloride at various concentrations were used as standards.

Thus, the desired dissolution rate of a sustained release dosage form of venlafaxine hydrochloride, impossible to achieve with hydrogel tablet technology, has been achieved with the film-coated spheroid compositons of this invention.

**Claims**

1.    An encapsulated, extended release formulation of venlafaxine hydrochloride comprising a hard gelatin capsule containing a therapeutically effective amount of spheroids comprised of venlafaxine hydrochloride, microcrystalline cellulose and hydroxypropyl methylcellulose coated with ethyl cellulose and hydroxypropylmethylcellulose.

2.    An extended release formulation according to claim I wherein the spheroids are composed of 37.3% by weight of venlafaxine hydrochloride, 0.5% by weight of hydroxypropylmethylcellulose 2208, and 62.17% by weight of microcrystalline cellulose.

3.    An extended release formulation according to claim 1 wherein the film coating is comprised of ethyl cellulose

6

(4.81% of total weight) and hydroxypropylmethylcellulose (0.85% of total weight).

4. An extended release formulation according to claim 1 wherein the film coating is comprised of ethyl cellulose (4.04% of total weight) and hydroxypropylmethylcellulose (0.714% of total weight).

5. An extended release formulation according to claim 1 wherein the film coating is comprised of ethyl cellulose (2.48% of total weight) and hydroxypropylmethylcellulose (0.437% of total weight).

6. An extended release formulation according to any one of claims 3 to 5 wherein the spheroids are composed of 37.3% by weight of venlafaxine hydrochloride, 0.5% by weight of hydroxypropylmethylcellulose 2208, and 62.17% by weight of microcrystalline cellulose.

7. An extended release formulation according to any one of claims I to 6 wherein the level of coating of the coated spheroid amounts to five to ten percent (w/w).

8. An extended release formulation according to any one of claims 1 to 6 wherein the level of coating of the coated spheroid amounts to 6 to 8 percent (w/w).

9. An extended release formulation of venlafaxine hydrochloride for once daily administration which comprises spheroids containing 37.3% venlafaxine, 62.17% microcrystalline cellulose and 0.5% hydroxypropylmethylcellulose type 2208, coated with a quantity of a mixture comprised of 15% ethyl cellulose type HG 2834 and 85% hydroxypropylmethylcellulose type 2910 sufficient to give coated spheroids having a dissolution profile which gives the desired release rate over a 24 hour period.

10. An extended release formulation of venlafaxine hydrochloride according to claim 9 which provides lower peak serum levels of up to 150 ng/ml and extended therapeutically effective plasma levels over a twenty four hour period.

11. A coated spheroid in which the spheroid is comprised of venlafaxine hydrochloride, microcrystalline cellulose and hydroxypropyl methylcellulose and is coated with ethyl cellulose and hydroxypropylmethylcellulose.

12. A coated spheroid according to claim 11 wherein the film coating is comprised of ethyl cellulose (4.81% of total weight) and hydroxypropylmethylcellulose (0.85% of total weight).

13. A coated spheroid according to claim 11 wherein the film coating is comprised of ethyl cellulose (4.04% of total weight) and hydroxypropylmethylcellulose (0.714% of total weight).

14. A coated spheroid according to claim 11 wherein the film coating is comprised of ethyl cellulose (2.48% of total weight) and hydroxypropylmethylcellulose (0.437% of total weight).

15. A coated spheroid according to any one of claims 11 to 14 wherein the spheroid is composed of 37.3% by weight of venlafaxine hydrochloride, 0.5% by weight of hydroxypropylmethylcellulose 2208, and 62.17% by weight of microcrystalline cellulose.

16. A coated spheroid according to any one of claims 11 to 15 wherein the level of coating of the coated spheroid amounts to five to ten percent (w/w).

17. A coated spheroid according to any one of claims 11 to 16 wherein the level of coating of the coated spheroid amounts to 6 to 8 percent (w/w).

18. A coated spheroid according to any one of claims 11 to 17 in which the coating is composed of ethyl cellulose (15% of total weight), having a 44.0-51.0% content of ethoxy groups, and hydroxypropylmethylcellulose (85% of total weight) having a methoxy content of 28.0-30.0% and a hydroxypropoxy group content of 7.0-12.0%.

19. A coated spheroid according to claim 11 comprising from 30 to 40 percent venlafaxine hydrochloride, from 50 to about 70 percent microcrystalline cellulose, from 0.25 to about 1 percent hydroxypropylmethylcellulose and from 5 to 10 percent film coating, all on a weight/ weight basis.

**Revendications**

1. Formulation encapsulée à libération prolongée de chlorhydrate de venlafaxine comprenant une gélule de gélatine dure contenant une quantité thérapeutiquement efficace de sphéroïdes constitués de chlorhydrate de venlafaxine, de cellulose microcristalline et d'hydroxypropylméthylcellulose enrobés d'éthylcellulose et d'hydroxypropylméthylcellulose.

2. Formulation à libération prolongée selon la revendication 1, dans laquelle les sphéroïdes sont constitués de 37,3% en poids de chlorhydrate de venlafaxine, de 0,5% en poids d'hydroxypropylméthylcellulose 2208 et de 62,17% en poids de cellulose microcristalline.

3. Formulation à libération prolongée selon la revendication 1, dans laquelle l'enrobage par film est constitué d'éthylcellulose (4,81% du poids total) et d'hydroxypropylméthylcellulose (0,85% du poids total).

4. Formulation à libération prolongée selon la revendication 1, dans laquelle l'enrobage par film est constitué d' éthylcellulose (4,04% du poids total) et d'hydroxypropylméthylcellulose (0,714% du poids total).

5. Formulation à libération prolongée selon la revendication 1, dans laquelle l'enrobage par film est constitué d'éthylcellulose (2,48% du poids total) et d'hydroxypropylméthylcellulose (0,437% du poids total).

6. Formulation à libération prolongée selon l'une quelconque des revendications 3 à 5, dans laquelle les sphéroïdes sont constitués de 37,3% en poids de chlorhydrate de venlafaxine, de 0,5% en poids d'hydroxypropylméthylcellulose 2208 et de 62,17% en poids de cellulose microcristalline.

7. Formulation à libération prolongée selon l'une quelconque des revendications 1 à 6, dans laquelle le niveau d'enrobage du sphéroïde enrobé est de cinq à dix pour cent (poids/poids).

8. Formulation à libération prolongée selon l'une quelconque des revendications 1 à 6, dans laquelle le niveau d'enrobage du sphéroïde enrobé est de 6 à 8 pour cent (poids/poids).

9. Formulation à libération prolongée de chlorhydrate de venlafaxine pour administration quotidienne unique, qui comprend des sphéroïdes contenant 37,3% de venlafaxine, 62,17% de cellulose microcristalline et 0,5% d'hydroxypropylméthylcellulose de type 2208, enrobés d'une quantité d'un mélange constitué de 15% d' éthylcellulose de type HG 2834 et de 85% d'hydroxypropylméthylcellulose de type 2910 suffisante pour obtenir des sphéroïdes enrobés ayant un profil de dissolution qui donne le taux de libération souhaité sur une période de 24 heures.

10. Formulation à libération prolongée de chlorhydrate de venlafaxine selon la revendication 9, qui fournit des niveaux inférieurs de sérum de crête jusqu'à 150 ng/ml et des niveaux de plasma thérapeutiquement efficaces prolongés sur une période de vingt-quatre heures.

11. Sphéroïde enrobé, dans lequel le sphéroïde est constitué de chlorhydrate de venlafaxine, de cellulose microcristalline et d'hydroxypropylméthylcellulose et est enrobé d'éthylcellulose et d'hydroxypropylméthylcellulose.

12. Sphéroïde enrobé selon la revendication 11, dans lequel l'enrobage par film est constitué d'éthylcellulose (4,81% du poids total) et d'hydroxypropylméthylcellulose (0,85% du poids total).

13. Sphéroïde enrobé selon la revendication 11, dans lequel l'enrobage par film est constitué d'éthylcellulose (4,04% du poids total) et d'hydroxypropylméthylcellulose (0,714% du poids total).

14. Sphéroïde enrobé selon la revendication 11, dans lequel l'enrobage par film est constitué d'éthylcellulose (2,48% du poids total) et d'hydroxypropylméthylcellulose (0,437% du poids total).

15. Sphéroïde enrobé selon l'une quelconque des revendications 11 à 14, dans lequel le sphéroïde est constitué de 37,3% en poids de chlorhydrate de venlafaxine, de 0,5% en poids d'hydroxypropylméthylcellulose 2208 et de 62,17% en poids de cellulose microcristalline.

16. Sphéroïde enrobé selon l'une quelconque des revendications 11 à 15, dans lequel le niveau d'enrobage du sphéroïde enrobé est de cinq à dix pour-cent (poids/poids).

17. Sphéroïde enrobé selon l'une quelconque des revendications 11 à 16, dans lequel le niveau d'enrobage du sphéroïde enrobé est de 6 à 8% (poids/poids).

18. Sphéroïde enrobé selon l'une quelconque des revendications 11 à 17, dans lequel l'enrobage est constitué d'éthylcellulose (15% du poids total), ayant une teneur en groupes éthoxy de 44,0 à 51,0%, et d'hydroxypropylméthylcellulose (85% du poids total), ayant une teneur en groupes méthoxy de 28,0 à 30,0% et une teneur en groupes hydroxypropoxy de 7,0 à 12,0%.

19. Sphéroïde enrobé selon la revendication 11, comprenant 30 à 40 pour-cent de chlorhydrate de venlafaxine, 50 à environ 70 pour-cent de cellulose microcristalline, 0,25 à 1 pour-cent d'hydroxypropylméthylcellulose et 5 à 10 pour-cent d'enrobage par film, le tout sur la base de poids/poids.

**Patentansprüche**

1. Eingekapselte Formulierung mit verlängerter Abgabe von Venlafaxin-hydrochlorid, welche eine harte Gelatinekapsel umfasst, welche eine therapeutisch wirksame Menge von Sphäroiden enthält, welche Venlafaxin-hydrochlorid, mikrokristalline Cellulose und Hydroxypropylmethylcellulose umfassen, überzogen mit Ethylcellulose und Hydroxypropylmethylcellulose.

2. Formulierung mit verlängerter Abgabe gemäß Anspruch 1, wobei die Sphäroide aus 37,3 Gew.-% Venlafaxin-hydrochlorid, 0,5 Gew.-% Hydroxypropylmethylcellulose 2208 und 62,17 Gew.-% mikrokristalliner Cellulose bestehen.

3. Formulierung mit verlängerter Abgabe gemäß Anspruch 1, wobei der Filmüberzug Ethylcellulose (4,81% des Gesamtgewichts) und Hydroxypropylmethylcellulose (0,85% des Gesamtgewichts) umfasst.

4. Formulierung mit verlängerter Abgabe gemäß Anspruch 1, wobei der Filmüberzug Ethylcellulose (4,04% des Gesamtgewichts) und Hydroxypropylmethylcellulose (0,714% des Gesamtgewichts) umfasst.

5. Formulierung mit verlängerter Abgabe gemäß Anspruch 1, wobei der Filmüberzug Ethylcellulose (2,48% des Gesamtgewichts) und Hydroxypropylmethylcellulose (0,437% des Gesamtgewichts) umfasst.

6. Formulierung mit verlängerter Abgabe gemäß einem der Ansprüche 3 bis 5, wobei die Sphäroide aus 37,3 Gew.-% Venlafaxin-hydrochlorid, 0,5 Gew.-% Hydroxypropylmethylcellulose 2208 und 62,17 Gew.-% mikrokristalliner Cellulose bestehen.

7. Formulierung mit verlängerter Abgabe gemäß einem der Ansprüche 1 bis 6, wobei sich der Überzugsgehalt des überzogenen Sphäroids auf fünf bis zehn Prozent (M/M) beläuft.

8. Formulierung mit verlängerter Abgabe gemäß einem der Ansprüche 1 bis 6, wobei sich der Überzugsgehalt des überzogenen Sphäroids auf 6 bis 8 Prozent (M/M) beläuft.

9. Formulierung mit verlängerter Abgabe von Venlafaxin-hydrochlorid für einmal tägliche Verabreichung, welche Sphäroide umfasst, welche 37,3% Venlafaxin, 62,17% mikrokristalline Cellulose und 0,5% Hydroxypropylmethylcellulose Typ 2208 enthalten, überzogen mit einer Menge eines Gemisches, welches 15% Ethylcellulose Typ HG 2834 und 85% Hydroxypropylmethylcellulose Typ 2910 umfasst, was ausreichend ist, um überzogene Sphäroide mit einem Auflösungsprofil zu ergeben, welches die gewünschte Abgaberate über einen 24 Stunden Zeitraum ergibt.

10. Formulierung mit verlängerter Abgabe von Venlafaxin-hydrochlorid gemäß Anspruch 9, welche Serumgehalte mit niedrigerem Peak bis zu 150 ng/ml und verlängerte therapeutisch wirksame Plasmagehalte über einen Zeitraum von vierundzwanzig Stunden vorsieht.

11. Überzogenes Sphäroid, in welchem das Sphäroid Venlafaxin-hydrochlorid, mikrokristalline Cellulose und Hydroxypropylmethylcellulose umfasst und mit Ethylcellulose und Hydroxypropylmethylcellulose überzogen ist.

12. Überzogenes Sphäroid gemäß Anspruch 11, wobei der Filmüberzug Ethylcellulose (4,81% des Gesamtgewichts) und Hydroxypropylmethylcellulose (0,85% des Gesamtgewichts) umfasst.

13. Überzogenes Sphäroid gemäß Anspruch 11, wobei der Filmüberzug Ethylcellulose (4,04% des Gesamtgewichts) und Hydroxypropylmethylcellulose (0,714% des Gesamtgewichts) umfasst.

14. Überzogenes Sphäroid gemäß Anspruch 11, wobei der Filmüberzug Ethylcellulose (2,48% des Gesamtgewichts) und Hydroxypropylmethylcellulose (0,437% des Gesamtgewichts) umfasst.

15. Überzogenes Sphäroid gemäß einem der Ansprüche 11 bis 14, wobei der Sphäroid aus 37,3 Gew.-% Venlafaxin-hydrochlorid, 0,5 Gew.-% Hydroxypropylmethylcellulose 2208 und 62,17 Gew.-% mikrokristalliner Cellulose besteht.

16. Überzogenes Sphäroid gemäß einem der Ansprüche 11 bis 15, wobei sich der Überzugsgehalt des überzogenen Sphäroids auf fünf bis zehn Prozent (M/M) beläuft.

17. Überzogenes Sphäroid gemäß einem der Ansprüche 11 bis 16, wobei sich der Überzugsgehalt des überzogenen Sphäroids auf 6 bis 8 Prozent (M/M) beläuft.

18. Überzogenes Sphäroid gemäß einem der Ansprüche 11 bis 17, in welchem der Überzug aus Ethylcellulose (15% des Gesamtgewichts) besteht, mit einem 44,0-51,0% Gehalt an Ethoxygruppen und Hydroxypropylmethylcellulose (85% Gesamtgewicht) mit einem Methoxygehalt von 28,0-30,0% und einem Hydroxypropoxygruppengehalt von 7,0-12,0%.

19. Überzogenes Sphäroid gemäß Anspruch 11, welches von 30 bis 40 Prozent Venlafaxin-hydrochlorid, von 50 bis etwa 70 Prozent mikrokristalline Cellulose, von 0,25 bis etwa 1 Prozent Hydroxypropylmethylcellulose und von 5 bis 10 Prozent Filmüberzug umfasst, alle auf einer Gewicht/Gewicht Basis.